# EUROPEAN PATENT APPLICATION

(11) **EP 4 745 975 A1**
(43) Date of publication of application: **20.05.2026**
(21) Application number: 25213550.4
(22) Date of filing: 05.11.2025
(51) Int. Cl.: G16C 20/50, G16C 20/30, G16C 20/70, G16C 20/90, G16C 20/40

(54) **SIMILARITY-DRIVEN GENERATION OF MOLECULES**

(30) Priority: 14.11.2024 US 202418948298
(71) Applicant: Microsoft Technology Licensing, LLC, Redmond, WA 98052 (US)
(72) Inventor: MILLS, Alexis Woodward, Redmond, 98052 (US); EFIMOVSKAYA, Alexandra, Redmond, 98052 (US); LIU, Hongbin, Redmond, 98052 (US); BAKER, Nathan Andrew, Redmond, 98052 (US); SUN, Chong, Redmond, 98052 (US)
(74) Representative: CMS Cameron McKenna Nabarro Olswang LLP

(57) **Abstract**

One example provides a method (200) of generating candidate molecules, the method comprising obtaining (202) input of a seed dataset comprising one or more seed chemical objects and inputting (206) the one or more seed chemical objects into a neural network to form one or more seed chemical object embeddings. The method further comprises performing (208) a vector-based chemical similarity search by comparing the one or more seed chemical object embeddings to a plurality of embeddings stored in a vector database to obtain a search result dataset comprising a plurality of search result chemical objects, each embedding of the plurality of embeddings comprising a vector representation of a chemical object. The method further comprises forming (212) an extended seed dataset based at least upon on the seed dataset and the search result dataset, and fine-tuning (216) a pretrained generative model using the extended seed dataset to form a fine-tuned generative model.

## Description

### BACKGROUND

In the field of chemical discovery, various computerized methods can be used to search for novel molecular structures with one or more desired properties associated with an intended application. Generative models for molecular structures are a class of computational models that use artificial intelligence (AI) to predict rational ways of combining molecular structure fragments to create novel molecular structures.

### SUMMARY

This Summary is provided to introduce a selection of concepts in a simplified form that are further described below in the Detailed Description. This Summary is not intended to identify key features or essential features of the claimed subject matter, nor is it intended to be used to limit the scope of the claimed subject matter. Furthermore, the claimed subject matter is not limited to implementations that solve any or all disadvantages noted in any part of this disclosure.

Examples are disclosed that relate to extending a seed dataset of chemical objects via a chemical similarity search, and using the extended seed dataset to fine-tune a generative model for molecular structures. One example provides a method of generating candidate molecules, the method comprising obtaining input of a seed dataset comprising one or more seed chemical objects corresponding to one or more seed molecules, and inputting the one or more seed chemical objects into a neural network to form one or more seed chemical object embeddings. The method further comprises performing a vector-based chemical similarity search by comparing the one or more seed chemical object embeddings to a plurality of embeddings stored in a vector database to obtain a search result dataset comprising a plurality of search result chemical objects, each embedding of the plurality of embeddings comprising a vector representation of a chemical object. The method further comprises forming an extended seed dataset based at least upon on the seed dataset and the search result dataset; and fine-tuning a pretrained generative model using the extended seed dataset to form a fine-tuned generative model.

### BRIEF DESCRIPTION OF THE DRAWINGS

FIG. 1 schematically shows an example of a computing architecture for implementing a searchable vector database and one or more generative models.
FIG. 2 shows a flow diagram of an example method for performing a chemical similarity search to form an extended seed dataset of chemical objects, and fine-tuning a generative model based on the extended seed dataset.
FIG. 3 shows a block diagram of an example computing system.

### DETAILED DESCRIPTION

As introduced above, the goal of chemical discovery is the identification of new molecules having one or more desired properties associated with an intended application. The chemical space for small molecules is large enough (~ 10⁶⁰) that brute force enumeration followed by filtering molecules for desired properties is impractical. Recently, generative models have been developed to design novel molecular structures in an intelligent manner that attempts to guide generation towards molecules that are likely to satisfy desired criteria. Generative models for molecular structures can provide a relatively efficient method of predicting the most rational way of combining various structure fragments (motif vocabulary) to generate novel molecular structures. However, the motif vocabulary is usually built upon heuristic rules and is typically fixed during generation. Such rules can cause difficulties in capturing common substructures from large amounts of molecules. On the other hand, small datasets can lead to overfitting where molecule generation tends to converge to a relatively narrow set of molecules that lack diversity. Additionally, it can be challenging to generate molecules that meet desired criteria.

Accordingly, examples are disclosed that relate to generative models that utilize similarity-driven guided generation of molecules based on chemical properties. The disclosed examples include performing a vector-based chemical similarity search using a seed dataset of chemical objects to form an extended seed dataset of chemical objects that can be used to fine-tune a generative model. Briefly, a relatively small set of one or more seed chemical objects corresponding to initial seed molecules is used as a basis to conduct a similarity search. The initial seed molecules can be selected based on one or more properties, such as chemical/physical properties related to an application. The similarity search can be performed using a neural network (NN) to generate an embedding for a seed molecule, and a vector database comprising stored embeddings. As described in more detail below, an embedding is a vector representation of a chemical object comprising embedded structural information and optionally embedded property information. Examples of chemical objects that can be represented via embeddings include molecules, materials, and/or crystal structures. The embeddings for the seed chemical objects are used to query the vector database and retrieve a search result dataset of chemical objects. The seed chemical objects and search result set of chemical objects are combined to form an extended seed dataset of chemical objects. Next, one or more pretrained generative models are fine-tuned using the extended seed dataset. Fine-tuning helps to guide the model towards generation of molecular structures of interest, i.e., molecules that have similar motifs and/or similar properties to molecules of the extended seed dataset. The fine-tuned generative models can then be used to generate candidate chemical objects corresponding to candidate molecules. The candidate molecules can be evaluated for novelty and/or a potential match to the desired criteria. Then, one or more candidate molecules can be selected for an intended application, such as an experimental trial synthesis. Use of fine-tuned generative models can identify molecules of interest more efficiently than other chemical discovery methods. In this manner, the disclosed examples can potentially provide cost and time savings in chemical discovery pipelines and synthesis trials.

Fine-tuning on a relatively small dataset can lead to problems, such as overfitting. In the task of novel molecule generation, overfitting can result in candidate molecules that lack diversity. In some examples, an initial seed dataset can comprise a relatively small number of chemical objects (e.g., 1-10) where overfitting becomes a concern. The disclosed examples can help avoid overfitting by using an extended seed dataset comprising a greater number of chemical objects (e.g., hundreds to sub-millions of chemical objects). By extending the seed dataset via a chemical similarity search, the output from the fine-tuned generative models can yield candidate molecules that are more diverse that molecules generated via other methods.

The disclosed examples can provide for property-driven conditional generation of candidate molecules with one or more desired properties. Conditional generation begins with a vector-based search to identify chemical objects that meet selected criteria. At a baseline level, the vector-based similarity search returns chemical objects with similar structures to the initial seed chemical object(s). Due to structural similarity, the chemical objects in the search result dataset have a substantial likelihood of having one or more properties similar to the initial seed chemical objects. The quality of the chemical similarity search can be improved by using a fine-tuned neural network to generate embeddings comprising embedded property information. This provides for a vector-based similarity search with property encoding to help return a broader set of chemical objects with similar structures and properties. In some examples, embeddings can comprise embedded property information for multiple properties. In some examples, a plurality of chemical similarity searches can be performed based on a corresponding plurality of selected properties. The search results can be merged to form an extended seed dataset of chemical objects that each satisfy one or more of the selected properties. As such, a generative model can be fine-tuned for multi-property conditional generation. In some examples, a pretrained generative model can be fine-tuned based on chemical objects matching a first property and then further fine-tuned based on chemical objects matching a second property.

Any suitable generative model(s) can be used. One example of a generative model that can be used for generating novel molecular structures is GraphBPE which uses molecular graphs to model molecules and applies a subword tokenization method in a data preprocessing step (Yuchen Shen and Barnabás Póczos. "GraphBPE: Molecular Graphs Meet Byte-Pair Encoding." arXiv preprint arXiv:2407.19039 (2024).). Another example of a generative model is MoLeR which uses an encoder/decoder model (Maziarz, Krzysztof, et al. "Learning to extend molecular scaffolds with structural motifs." arXiv preprint arXiv:2103.03864 (2021).). Generative models are pretrained using a large dataset. One example of a publicly accessible dataset of chemical objects is PubChem, available at pubchem.ncbi.nlm.nih.gov (Kim S, Chen J, Cheng T, et al. PubChem 2023 update. Nucleic Acids Res. 2023;51(D1):D1373-D1380.). Generative models can be pretrained using any suitable training algorithm(s), including gradient descent (e.g. stochastic gradient descent) with backpropagation. After pretraining the generative models, the pretrained generative model is fine-tuned using the extended seed dataset. The fine-tuning process helps to bias output from the generative model towards candidate molecules that are similar to the seed molecules of the extended seed dataset.

The generative models employ motifs (structure fragments) in generating novel molecular structures. Motifs help in the generation of semantically sensible molecular structures and avoid common pitfalls, such as partially filled rings. Typically, predefined motifs are employed when fine-tuning a generative model using a relatively small seed dataset. However, the disclosed examples provide for fine-tuned generative models that learn the motifs of the extended seed dataset and avoids predefined motifs. Avoiding predefined motifs help to make the generation more agile and can help to explore the relevant chemical space more efficiently than other methods.

In various examples, an assembly of generative models can be used. For example, a plurality of pretrained generative models can each be fine-tuned using the extended seed dataset of chemical objects. In some examples, different pretrained generative models can be fine-tuned using different seed datasets. Candidate chemical objects generated from the various fine-tuned generative models can be combined and output to a user.

After generating candidate chemical objects, various filtering techniques can be employed based on any suitable criteria. Examples include filtering based on novelty, physical properties, and chemical properties. For example, the candidate chemical objects can be compared to a chemical database to search for, and remove, non-novel chemical objects. Additionally, various computational tools can be used to predict properties of the candidate chemical objects, which can then be filtered based on one or more selected property values. Examples of computational tools include AI models, cheminformatics tools, and quantum chemistry calculations. Chemical objects that have a predicted property value outside the selected range can be removed from the set of candidate chemical objects. Further examples of filtering include filtering based on environmental regulations, commercial regulations, and synthesizability. One or more candidate chemical objects can be selected for an intended use, such as experimental trial synthesis. Synthesized chemicals can then be characterized and evaluated for performance in a chemical application.

Candidate chemical objects generated via the fine-tuned generative models can be used to further improve the chemical discovery pipeline. In some examples, candidate chemical objects can be added to the chemical database for possible usage in forming a subsequent extended seed dataset. In some examples, candidate chemical objects may be used to fine-tune the generative models. For example, chemical objects can be checked for novelty. Then, the fine-tuned generative model is further fine-tuned using the novel chemical objects. As novel chemical objects can add a new motif vocabulary, the chemical discovery workflow can be iterated to enrich the diversity of the next generation of candidate molecules.

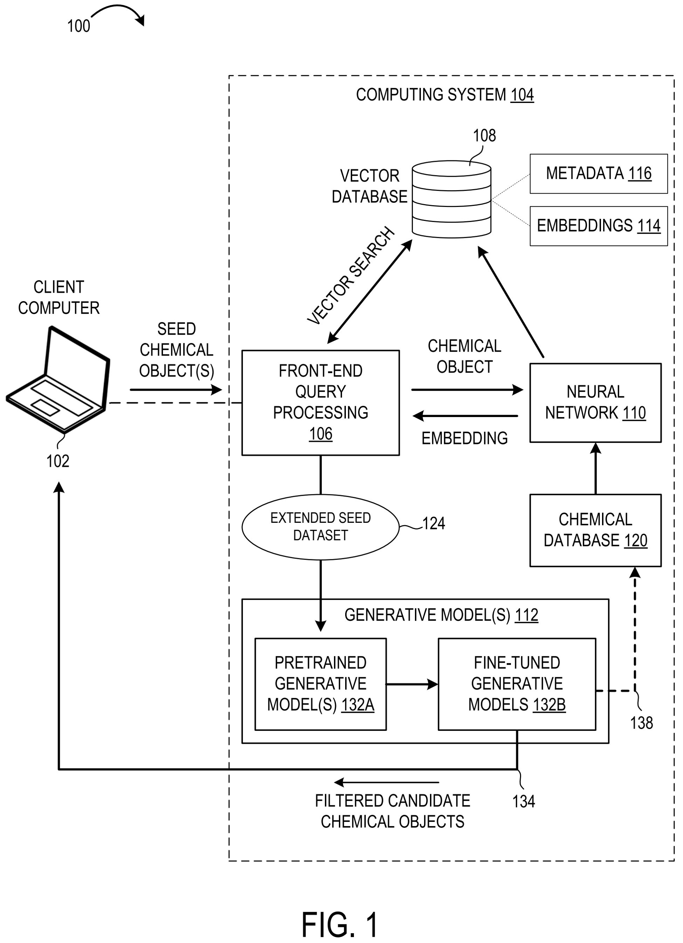

FIG. 1 schematically shows an example of a computing architecture 100 for processing a chemical discovery query and returning candidate chemical objects to a user based on the chemical discovery query. A chemical discovery query can comprise an initial seed dataset comprising one or more seed chemical objects. A chemical object can include any suitable chemical structure information, such as cartesian coordinates of atoms in a molecule and/or bonding information for a molecule. Examples of inputs of chemical structure information include text strings, such as a chemical name or a simplified molecular-input line-entry system (SMILES) string. Additional examples include 3-dimensional (3D) structural data with connectivity information (e.g., XYZ format, crystallographic information file (CIF) format, MOL format, Protein Data Bank (PDB) format, etc.).

A chemical discovery query further can comprise property information related to one or more selected properties. Examples of property information include numerical values, numerical ranges, and classifications for a chemical or physical property. A chemical discovery query further can include criteria for filtering generated chemical objects. Filtering is discussed in more detail below. A chemical discovery query further can include a number of candidate chemical objects to generate and/or a requested number of results to output. A chemical discovery query further can include a selected size of an extended dataset, a multi-stage fine-tuning protocol, as well as any other suitable information related to the chemical discovery workflow.

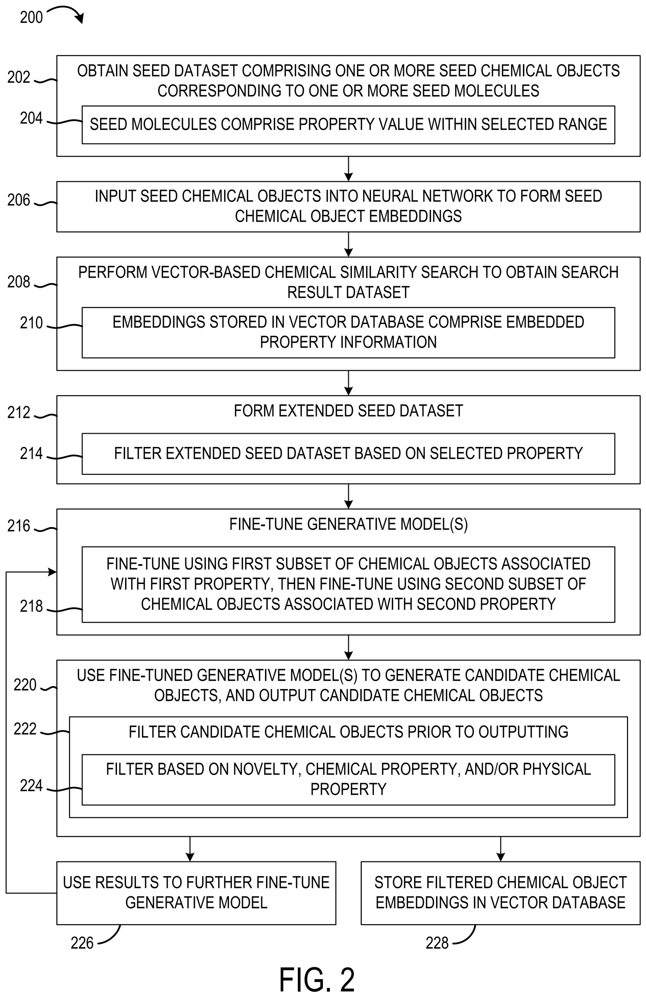

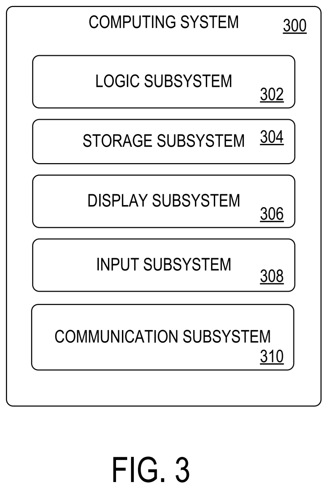

As illustrated in FIG. 1, a client computer 102 can submit a chemical discovery query to a computing system 104 and receive output of candidate chemical objects from computing system 104. Computing system 104 comprises one or more processors configured to perform vector-based chemical similarity searches, fine-tune generative models, and perform various other functions. An example method of processing chemical discovery queries is discussed below with regard to FIG. 2. Computing system 104 can represent a data center in some examples. Examples of computing systems are described in more detail below with regard to FIG. 3.

Computing system 104 comprises a front-end module 106 for chemical discovery query processing. Computing system 104 further comprises a storage system storing data for a vector database 108, one or more neural networks 110, and generative models 112. Vector database 108 comprises a plurality of embeddings 114 for a respective plurality of chemical objects, including molecules and/or materials (e.g., crystal structures or other representation of solids). Each embedding comprises a vector representation of the structure of a chemical object. The vector database further comprises metadata 116. Metadata 116 can comprise any suitable information for chemical objects corresponding to embeddings 114. In some examples, metadata 116 can include, for each embedding, a chemical object identification (ID) corresponding to the embedding. Examples of metadata include chemical names, chemical formulas, and ID numbers. In some examples, vector database 108 can optionally comprise property data for one or more chemical objects corresponding to embeddings 114. Examples of properties include a formation energy, a boiling point, a flash point, an ionization energy, a bandgap, and a dielectric constant. Further examples include classifications, such as metal vs. non-metal or conducting vs. insulating. In various examples, the property data can comprise experimental properties and/or predicted properties. However, property data may not exist for some chemical objects.

Neural network 110 is configured to generate embeddings for chemical objects. Examples of neural networks include transformers, graph-transformers, convolutional neural networks (CNNs), and/or GNNs. GNNs are trained to perform inference on data described by a mathematical graph. Graphs can be a suitable choice for representing a chemical object, such as a molecule, where nodes represent atoms and edges represent bonds. Examples of GNNs suitable for generating embeddings include GNNs trained using the Graphormer deep learning package (Ying, Chengxuan, et al. "Do transformers really perform badly for graph representation?." Advances in Neural Information Processing Systems 34 (2021): 28877-28888.). Any suitable configuration can be used for neural network 110. In some examples, neural network 110 comprises a GNN configured with ≥ 6 layers. In some examples, neural network 110 comprises ≥ 80 hidden dimensions. In some more specific examples, neural network 110 comprises 12 layers, 32 attention heads, and 24 hidden dimensions for each attention head. In other examples, any other suitable configuration can be used.

Neural network 110 can be trained using any suitable training data. Computing system 104 optionally comprises a chemical database 120 that can be used to provide training data. Chemical database 120 can comprise one or more sets of chemical objects. In some examples, chemical database 120 comprises labeled training data comprising structural information and property information for each chemical object of a set of chemical objects. The property information can comprise values for one or more properties (e.g., boiling point). The labeled training data can be used in a fine-tuning process as described below. Additionally or alternatively, a third-party chemical database can be used to provide training data for training neural network 110. One example of a publicly accessible third-party chemical database is PubChem, available at pubchem.ncbi.nlm.nih.gov (Kim S, Chen J, Cheng T, et al. PubChem 2023 update. Nucleic Acids Res. 2023;51(D1):D1373-D1380.).

Neural network 110 can be trained using supervised training or unsupervised training. In supervised training, labeled training data is input into neural network 110 and the neural network is trained to predict a property, such as an energy. In some examples, a computationally inexpensive energy calculation can be used to predict energies for use in the supervised training process. In other examples, any other suitable predicted property can be used. In further example, empirical data can be used. Supervised training can be performed using any suitable training algorithm(s), including gradient descent (e.g. stochastic gradient descent) with backpropagation.

Alternatively, neural network 110 can be trained using unsupervised training. In unsupervised training, the neural network can be trained to predict structural information of a chemical object. For example, one or more atoms of a chemical object can be masked, and the neural network learns to predict a type and/or location of the masked atom in the chemical object.

After training, neural network 110 can be used to generate embeddings based on chemical structures. As discussed above, an embedding comprises a vector representation of a chemical structure. Embeddings can capture intricate properties and relationships of chemical structures in a multidimensional space.

To generate an embedding, a chemical structure is input into the trained neural network 110, and the embedding for the chemical structure is output by the embedding layer. For example, a plurality of chemical objects from chemical database 120 and/or a third-party chemical database can be input into neural network 110 to generate embeddings. In some examples, a chemical database can be supplied by a user (e.g., from client computer 102). The embedding comprises a high-dimensional vector representation of the chemical structure, wherein the vector representation is formed from coefficients output by the embedding layer of the trained neural network. In some examples, the length of the vector corresponds to the number of hidden dimensions in the neural network. The generated embeddings are stored in vector database 108. Embeddings 114 are stored with metadata 116 comprising information identifying the chemical objects corresponding to the embeddings. Examples of metadata include a chemical object ID (e.g., CAS number) and a chemical formula.

Additionally, after initial training (pre-training) using structural data, neural network 110 can be fine-tuned. A fine-tuning process can include inputting labeled training data into a pretrained version of training neural network 110 to predict a chemical or physical property. Examples of properties include a boiling point, a flash point, an ionization energy, a bandgap, a dielectric constant, and classifications, such as metal vs. non-metal or conducting vs. insulating. After fine-tuning, neural network 110 can be used to generate embeddings for storage in vector database 108. Embeddings generated by a fine-tuned version of neural network 110 comprise embedded structural information and embedded property information. This allows for a vector-based search to identify chemical objects that are structurally similar and have similar property values to the target chemical object. As property data may not exist for some of the chemical objects stored in the vector database, the vector-based search can help provide chemical similarity search functionality that is not feasible using other search methods. In some examples, fine-tuning can be performed using user data. For example, client computer 102 can transmit labeled training data to computing system 104 to be used in the fine-tuning process. In some such examples, the fine-tuning process can be used to form a user version of vector database 108. This can allow a user to form a customized vector database without affecting a public version of vector database 108. In other examples, any other suitable labeled training data can be used.

Compared to training from scratch, fine-tuning can leverage prior understanding of chemistry captured by the pretrained model. This helps the fine-tuned model represent structural and functional characteristics with greater accuracy than a model trained from scratch. Further, the fine-tuning process can leverage the large training set used for the pretrained neural network and fine-tunes it using a relatively smaller dataset of labeled training data. As such, a pretrained neural network can be fine-tuned in a robust and cost-efficient manner.

In addition to forming embeddings for storage in vector database 108, neural network 110 can be used to generate an embedding that can be used in a chemical similarity search. To query the vector database, a seed chemical object is input into the trained neural network. The seed chemical object can include, e.g., text strings and 3D structural data, such as those listed above. The trained neural network generates a seed embedding comprising a vector representation of the seed chemical object. The seed embedding is used to query the vector database to identify other chemical objects that are structurally similar to the seed chemical object. As described in more detail below, the structural similarity of two chemical objects can be quantified based on the respective embeddings for the chemical objects. In this manner, one or more chemical objects that are structurally similar to the seed chemical object can be retrieved from vector database 108. In examples where a fine-tuned neural network is used, the seed embedding comprises embedded structural information and embedded property information for the seed chemical object. This allows for a vector-based search to identify chemical objects that are structurally similar and have similar property values to the seed chemical object. Embeddings can capture complex molecular characteristics and intricate relationships between chemicals in a multidimensional space. In this manner, embeddings can be more nuanced than other solutions that utilize rule-based algorithms or fingerprint methods for structural comparisons between different chemical structures. As such, a vector search of vector database 108 can provide a chemical similarity search with greater accuracy and more meaningful results than other search methods.

The vector-based chemical similarity search can employ a similarity score, for example. Any suitable similarity score can be used, such as a Euclidean distance, a Manhattan distance, or a cosine similarity. A relatively high degree of similarity between two embeddings indicates a relatively high degree of topological similarity between the two chemical objects corresponding to the embeddings. Thus, based on the embedding of the target chemical object and a similarity score metric, the vector search can retrieve a set of K similar chemical objects from the vector database. In examples where a fine-tuned neural network is used to generate embeddings, the embedding captures structural and functional characteristics within a single vector.

A vector database can offer improvements over traditional cheminformatics databases that are not optimized for high-dimensional data produced by deep-learning models. By using a vector database rather than a rule-based search method, querying can be more efficient than other methods. Additionally, the vector database can be configured for storing embeddings and high-efficiency querying of embeddings. The computational efficiency of the method also can surpass traditional algorithms (e.g., rule-based algorithms or fingerprint methods), as the embedding process condenses complex information into a manageable form. By using a high-dimensional vector representation of complex information, the system facilitates fast, scalable searches within the vector database. In some examples, 100 million entries can be searched in less than 0.1 seconds. Further, by using deep learning for embeddings, the system can capture complex molecular characteristics that other methods can overlook. This helps provide chemical similarity searches with greater accuracy and more meaningful results than other search methods. This can be useful in various applications, such as drug discovery, material design, and environmental studies.

Additionally, the use of fine-tuned neural network-based embeddings with embedded property information allows for a more nuanced representation of chemical objects, capturing structural intricacies and functional characteristics within a single vector. This dual representation is a significant advancement over methods that consider structural or property data in isolation. Additionally, by extracting embeddings from advanced neural network architectures, for example, fine-tuned Graph Neural Networks (GNNs), the system can discern subtle chemical and physical relationships that are often overlooked by conventional algorithms. This leads to more accurate and relevant search results, such as identifying viable alternatives for environmentally or health-sensitive chemicals. This efficiency, combined with the scalability, provides a valuable tool for rapid and precise chemical exploration and innovation.

Referring again to FIG. 1, the vector-based similarity search can return a search result dataset of chemical objects from vector database 108 based on a similarity score to one or more seed chemical objects of the seed dataset. The search result dataset is combined with the seed dataset to form an extended seed dataset 124. As mentioned above, in examples where a fine-tuned neural network is used to form embeddings comprising embedded property information, the vector-based chemical similarity search may be more effective at retrieving search result chemical objects that have similar properties to the seed chemical objects.

In some examples, extended seed dataset 124 is filtered prior to fine-tuning a generative model. For example, extended seed dataset 124 may be filtered based on the selected property associated with the initial seed chemical objects. As one illustrative example, a chemical discovery search may be targeted at identifying molecules with boiling points above a target temperature. In this example, the extended seed dataset can be filtered to remove molecules with boiling points below the target temperature. As another example, extended seed dataset 124 can be filtered to remove potentially toxic molecules (e.g., arsenic-containing molecules) in environmental applications. In further examples, any suitable filtering protocol can be performed.

After optional filtering, extended seed dataset 124 is used as input to fine-tune one or more pretrained generative models 132A to form a corresponding one or more fine-tuned generative models 132B. As discussed above, pretrained generative models 132A comprise generic generative models configured to generate stable candidate chemical objects unconditionally. The fine-tuning process further trains the pretrained generative models 132A to generate candidate chemical objects based on the motifs learned from chemical objects of extended seed dataset 124.

After fine-tuning, the one or more fine-tuned generative models 132B can be used to generate any suitable number N of candidate chemical objects. In various examples, N can be a predetermined number of candidate chemical objects or provided by a user (e.g., provided in a chemical discovery query).

After generating a suitable number of candidate chemical objects, a post-generation screening process can be performed to filter the set of candidates based on various criteria to form filtered output. Candidate chemical objects that meet the selected criteria are included in the filtered output while candidate chemical objects that do not meet the selected criteria are not included in filtered output. Filtering can be performed based on various criteria. For example, the candidate chemical objects can be checked for novelty with respect to the extended seed dataset. Chemical objects that are not novel can be removed from the set of candidate chemical objects. Filtering also can be performed based on properties. As empirical data for properties is not available for novel molecules, various computational tools can be employed to predict properties of the candidate chemical objects. Examples of computational tools include AI models, cheminformatics tools, and quantum chemistry calculations. For example, after predicting a value of a selected property for a candidate chemical object, the candidate chemical object can be removed based upon a predicted property value not meeting selected criteria for the selected property. Alternatively, the candidate chemical object can be included in filtered output based upon meeting the selected criteria. Examples of properties used for filtering include those listed above. Further examples include bond types, environmental regulations, reactivity, and synthesizability. In some examples, candidate chemical objects are filtered based at least upon the selected property used to conduct the vector-based similarity search. Additionally or alternatively, in some examples, candidate chemical objects are filtered based at least upon the selected property used to filter extended seed dataset 124.

After filtering, the candidate chemical objects can be output to client computer 102, as indicated at 134. As mentioned above, a user can select one or more candidate chemical objects for an intended use, such as an experimental trial synthesis. Additionally or alternatively, filtered candidate chemical objects can be output to chemical database 120, as indicated at 138. As such, novel candidate chemical objects can be input into neural network 110 and added to vector database 108. In this manner, novel chemical objects generated by fine-tuned generative model(s) 132B can be used to form an extended seed dataset in a subsequent chemical discovery query. Further, in some examples, candidate chemical objects may be used to further fine-tune the generative models. As novel chemical objects can add a new motif vocabulary, the chemical discovery workflow can be iterated to enrich the diversity of the next generation of candidate molecules. In this manner, generated candidate chemical objects can help improve the chemical discovery pipeline.

FIG. 2 shows a flow diagram of an example method 200 for performing a chemical discovery query including a chemical similarity search based on initial seed molecules and fine-tuning a generative model based on an extended seed dataset. Computing architecture 100 is an example of a computing architecture for processing chemical discovery queries using method 200.

At 202, method 200 comprises obtaining a seed dataset comprising one or more seed chemical objects corresponding to one or more seed molecules. Examples of chemical objects include text strings and 3D structural data. A text string representation can comprise any suitable information identifying a chemical object or describing bonding within a chemical object. Examples of text string representations for a chemical object include a chemical name, a chemical formula, and a SMILES string. Examples of 3D structural data include data in XYZ format, CIF format, MOL format, and PDB format. In some examples, at 204, each of the one or more seed molecules comprises a value of a selected property within a selected range. This can help bias the results of the chemical discovery process to generate candidate molecules comprising property values within the selected range.

At 206, method 200 comprises inputting the seed chemical objects into a trained neural network to form seed chemical object embeddings. As described above, an embedding is a vector representation of a chemical object comprising embedded structural information. In some examples, the trained neural network is a fine-tuned neural network, and the seed chemical object embeddings comprise embedded property information. In some such examples, the embedded property information comprises embedded property information for the selected property.

Continuing, at 208, method 200 comprises performing a vector-based chemical similarity search to obtain a search result dataset. The vector-based chemical similarity search comprises comparing the one or more seed chemical object embeddings to a plurality of embeddings stored in a vector database (e.g., vector database 108). The search result dataset comprises a plurality of search result chemical objects. In some examples, at 210, the plurality of embeddings stored in the vector database comprise embedded property information. In some examples, performing the vector-based similarity search comprises determining a similarity score between the one or more seed chemical object embeddings to an embedding in the vector database. Examples of similarity scores include a Euclidean distance, a Manhattan distance, and a cosine similarity. In some examples, the vector-based chemical similarity search can return a predetermined number of chemical objects or a user-selected number of chemical objects. In some examples, the vector-based chemical similarity search can return chemical objects within a similarity score threshold.

At 212, method 200 comprises forming an extended seed dataset (e.g., extended seed dataset 124) based at least upon the seed dataset and the search result dataset. In some examples, the extended seed dataset comprises two or more subsets of chemical objects, where each subset comprises chemical objects that meet a different set of criteria. For example, the extended seed dataset may comprise a first subset of chemical objects that meet selected criteria associated with a first property, and a second subset of chemical objects that meet selected criteria associated with a second property. In some examples, at 214, method 200 comprises filtering the extended seed dataset based on one or more selected properties. Filtering at 214 can comprise, for example, removing search result chemical objects corresponding to molecules comprising a value outside the selected range for the selected property.

Continuing, at 216, method 200 comprises fine-tuning a pretrained generative model (e.g., pretrained generative model(s) 132A) using the extended seed dataset to form a fine-tuned generative model (e.g., fine-tuned generative model(s) 132B). In some examples, fine-tuning can be done on the entire generative model. In some examples, one or more layers of the model are frozen, and fine-tuning is done on a subset of layers. Further, in some examples, a generative model can be augmented with one or more adapter layers (e.g., a task-specific layer trained from scratch). In some examples, step 216 comprises fine-tuning a plurality of pretrained generative models to form a respective plurality of fine-tuned generative models. Different generative models can be fine-tuned using different datasets of chemical objects, in some examples.

Additionally, in some examples, the fine-tuning process can comprise two or more fine-tuning stages. In some examples, at 218, the fine-tuning process comprises fine-tuning the generative model using a first subset of chemical objects associated with a first property to form an intermediate generative model. Then, the intermediate generative model is fine-tuned using a second subset of chemical objects associated with a second property to obtain the fine-tuned generative model. In some examples, fine-tuning can be performed in a manner similar to the original training.

Continuing, at 220, method 200 comprises using the one or more fine-tuned generative models to generate a plurality of candidate chemical objects and output the plurality of candidate chemical objects (e.g., output to client computer 102). In some examples, at 222, method 200 comprises filtering the plurality of candidate chemical objects to form a filtered plurality of chemical objects, and outputting the filtered plurality of chemical objects. In some examples, at 224, step 222 comprises filtering the plurality of candidate chemical objects based upon one or more of novelty, a chemical property, or a physical property.

In some examples, at 226, method 200 comprises performing further fine-tuning of the generative model using the filtered plurality of candidate chemical objects to form a further fine-tuned generative model. In some such examples, method 200 may further comprise generating additional candidate chemical objects using the further fine-tuned generative model. In some examples, at 228, method 200 comprises forming one or more embeddings for the filtered plurality of chemical objects and storing the one or more embeddings in the vector database.

Further, in some examples, method 200 comprises selecting one or more candidate chemical objects for experimental trial synthesis. Synthesized chemicals can be characterized to measure properties. Additionally, synthesized chemicals can be evaluated for performance in an intended chemical application. In other examples, one or more candidate chemical objects can be selected for any other intended use.

Thus, the disclosed example chemical discovery workflows provide for property-driven conditional generation of candidate molecules with one or more desired properties. The chemical discovery query includes conducting a vector-based chemical similarity search based at least upon a seed dataset of seed chemical objects to form an extended seed dataset. By using embeddings that encapsulate structural and functional characteristics of molecules, the vector-based chemical similarity search can efficiently identify chemical alternatives that share structure and/or properties with one or more seed chemical objects. The disclosed examples further provide for fine-tuning a pretrained generative model using the extended seed dataset. By using an extended seed dataset in fine-tuning, the disclosed examples can help avoid convergence problems and overfitting. As a result, the output candidate molecules are more diverse that molecules generated via other methods. Further, by selecting seed molecules with selected properties, the disclosed examples help guide the generative models toward generation of molecules comprising the selected properties.

In some embodiments, the methods and processes described herein may be tied to a computing system of one or more computing devices. In particular, such methods and processes may be implemented as a computer-application program or service, an application-programming interface (API), a library, and/or other computer-program product.

FIG. 3 schematically shows a non-limiting embodiment of a computing system 300 that can enact one or more of the methods and processes described above. Computing system 300 is shown in simplified form. Computing system 300 may take the form of one or more personal computers, server computers, tablet computers, home-entertainment computers, network computing devices, mobile computing devices, mobile communication devices (e.g., smart phone), and/or other computing devices.

Computing system 300 includes a logic subsystem 302 and a storage subsystem 304. Computing system 300 may optionally include a display subsystem 306, input subsystem 308, communication subsystem 310, and/or other components not shown in FIG. 3.

Logic subsystem 302 includes one or more physical devices configured to execute instructions. For example, the logic machine may be configured to execute instructions that are part of one or more applications, services, programs, routines, libraries, objects, components, data structures, or other logical constructs. Such instructions may be implemented to perform a task, implement a data type, transform the state of one or more components, achieve a technical effect, or otherwise arrive at a desired result.

The logic machine may include one or more processors configured to execute software instructions. Additionally or alternatively, the logic machine may include one or more hardware or firmware logic machines configured to execute hardware or firmware instructions. Processors of the logic machine may be single-core or multi-core, and the instructions executed thereon may be configured for sequential, parallel, and/or distributed processing. Individual components of the logic machine optionally may be distributed among two or more separate devices, which may be remotely located and/or configured for coordinated processing. Aspects of the logic machine may be virtualized and executed by remotely accessible, networked computing devices configured in a cloud-computing configuration.

Storage subsystem 304 includes one or more physical devices configured to hold instructions executable by the logic machine to implement the methods and processes described herein. When such methods and processes are implemented, the state of storage subsystem 304 may be transformed-e.g., to hold different data.

Storage subsystem 304 may include removable and/or built-in devices. Storage subsystem 304 may include optical memory (e.g., CD, DVD, HD-DVD, Blu-Ray Disc, etc.), semiconductor memory (e.g., RAM, EPROM, EEPROM, etc.), and/or magnetic memory (e.g., hard-disk drive, floppy-disk drive, tape drive, MRAM, etc.), among others. Storage subsystem 304 may include volatile, nonvolatile, dynamic, static, read/write, read-only, random-access, sequential-access, location-addressable, file-addressable, and/or content-addressable devices.

It will be appreciated that storage subsystem 304 includes one or more physical devices. However, aspects of the instructions described herein alternatively may be propagated by a communication medium (e.g., an electromagnetic signal, an optical signal, etc.) that is not held by a physical device for a finite duration.

Aspects of logic subsystem 302 and storage subsystem 304 may be integrated together into one or more hardware-logic components. Such hardware-logic components may include field-programmable gate arrays (FPGAs), program- and application-specific integrated circuits (PASIC / ASICs), program- and application-specific standard products (PSSP / ASSPs), system-on-a-chip (SOC), and complex programmable logic devices (CPLDs), for example.

The terms "module," "program," and "engine" may be used to describe an aspect of computing system 300 implemented to perform a particular function. In some cases, a module, program, or engine may be instantiated via logic subsystem 302 executing instructions held by storage subsystem 304. It will be understood that different modules, programs, and/or engines may be instantiated from the same application, service, code block, object, library, routine, API, function, etc. Likewise, the same module, program, and/or engine may be instantiated by different applications, services, code blocks, objects, routines, APIs, functions, etc. The terms "module," "program," and "engine" may encompass individual or groups of executable files, data files, libraries, drivers, scripts, database records, etc.

It will be appreciated that a "service", as used herein, is an application program executable across multiple user sessions. A service may be available to one or more system components, programs, and/or other services. In some implementations, a service may run on one or more server-computing devices.

When included, display subsystem 306 may be used to present a visual representation of data held by storage subsystem 304. This visual representation may take the form of a graphical user interface (GUI). As the herein described methods and processes change the data held by the storage machine, and thus transform the state of the storage machine, the state of display subsystem 306 may likewise be transformed to visually represent changes in the underlying data. Display subsystem 306 may include one or more display devices utilizing virtually any type of technology. Such display devices may be combined with logic subsystem 302 and/or storage subsystem 304 in a shared enclosure, or such display devices may be peripheral display devices.

When included, input subsystem 308 may comprise or interface with one or more user-input devices such as a keyboard, mouse, touch screen, or game controller. In some embodiments, the input subsystem may comprise or interface with selected natural user input (NUI) componentry. Such componentry may be integrated or peripheral, and the transduction and/or processing of input actions may be handled on- or off-board. Example NUI componentry may include a microphone for speech and/or voice recognition; an infrared, color, stereoscopic, and/or depth camera for machine vision and/or gesture recognition; a head tracker, eye tracker, accelerometer, and/or gyroscope for motion detection and/or intent recognition; as well as electric-field sensing componentry for assessing brain activity. Input subsystem 308 may be configured to capture natural language input (text or speech) from a user. Computing system 300 further may be configured to implement one or more natural language processing (NLP) models (e.g., a large language model (LLM)) to process data encoded in natural language input.

When included, communication subsystem 310 may be configured to communicatively couple computing system 300 with one or more other computing devices. Communication subsystem 310 may include wired and/or wireless communication devices compatible with one or more different communication protocols. As non-limiting examples, the communication subsystem may be configured for communication via a wireless telephone network, or a wired or wireless local- or wide-area network. In some embodiments, the communication subsystem may allow computing system 300 to send and/or receive messages to and/or from other devices via a network such as the Internet.

Another example provides a method of generating candidate molecules. The method comprises obtaining input of a seed dataset comprising one or more seed chemical objects corresponding to one or more seed molecules, and inputting the one or more seed chemical objects into a neural network to form one or more seed chemical object embeddings. The method further comprises performing a vector-based chemical similarity search by comparing the one or more seed chemical object embeddings to a plurality of embeddings stored in a vector database to obtain a search result dataset comprising a plurality of search result chemical objects, each embedding of the plurality of embeddings comprising a vector representation of a chemical object. The method further comprises forming an extended seed dataset based at least upon on the seed dataset and the search result dataset and fine-tuning a pretrained generative model using the extended seed dataset to form a fine-tuned generative model. In some such examples, the method further comprises using the fine-tuned generative model to generate a plurality of candidate chemical objects, and outputting the plurality of candidate chemical objects. Additionally or alternatively, in some such examples, fine-tuning the pretrained generative model comprises fine-tuning two or more pretrained generative models to form a corresponding two or more fine-tuned generative models, and using the fine-tuned generative model comprises using the two or more fine-tuned generative models to generate the plurality of candidate chemical objects. Additionally or alternatively, in some such examples, the method further comprises filtering the plurality of candidate chemical objects based on one or more of novelty, a chemical property, or a physical property to form filtered plurality of candidate chemical objects. Additionally or alternatively, in some such examples, the method further comprises performing further fine-tuning of the fine-tuned generative model using the filtered plurality of candidate chemical objects to form a further fine-tuned generative model, and using the further fine-tuned generative model to generate additional candidate chemical objects. Additionally or alternatively, in some such examples, the method further comprises storing an embedding of at least one filtered candidate chemical object of the filtered plurality of candidate chemical objects in the vector database. Additionally or alternatively, in some such examples, each of the seed molecules comprises a value of a selected property within a selected range. Additionally or alternatively, in some such examples, the method further comprises filtering the extended seed dataset to remove search result chemical objects corresponding to molecules comprising values outside the selected range for the selected property prior to fine-tuning the pretrained generative model. Additionally or alternatively, in some such examples, the extended seed dataset comprises a first subset of chemical objects that match a first property and a second subset of chemical objects that match a second property, and fine-tuning the pretrained generative model comprises fine-tuning the pretrained generative model using the first subset of chemical objects to obtain an intermediate generative model, and fine-tuning the intermediate generative model using the second subset of chemical objects to obtain the fine-tuned generative model. Additionally or alternatively, in some such examples, the embeddings stored in the vector database comprises embedded property information.

Another example provides a computing system implementing a neural network configured to form embeddings of chemical objects, a vector database storing embeddings of chemical objects, and one or more generative models. The computing system comprises a logic subsystem, and a storage subsystem comprising instructions executable by the logic subsystem to input one or more seed chemical objects into the neural network to form one or more seed chemical object embeddings. The instructions are further executable to perform a vector-based chemical similarity search by comparing the one or more seed chemical object embeddings to a plurality of embeddings stored in the vector database to obtain a plurality of search result chemical objects, each embedding of the plurality of embeddings comprising a vector representation of a chemical object, form an extended seed dataset based at least upon on the one or more seed chemical objects and the search result chemical objects, and fine-tune a pretrained generative model using the extended seed dataset to form a fine-tuned generative model. In some such examples, the instructions are further executable to use the fine-tuned generative model to generate a plurality of candidate chemical objects, and output the plurality of candidate chemical objects. Additionally or alternatively, in some such examples, the instructions executable to fine-tune the pretrained generative model are executable to fine-tune two or more pretrained generative models to form a corresponding two or more fine-tuned generative models, and the instructions are further executable to use the two or more fine-tuned generative models to generate the plurality of candidate chemical objects. Additionally or alternatively, in some such examples, the instructions are further executable to filter the plurality of candidate chemical objects based on one or more of novelty, a chemical property, or a physical property to form filtered plurality of candidate chemical objects. Additionally or alternatively, in some such examples, the instructions are further executable to perform further fine-tuning of the fine-tuned generative model using the filtered plurality of candidate chemical objects to form a further fine-tuned generative model, and use the further fine-tuned generative model to generate additional candidate chemical objects. Additionally or alternatively, in some such examples, the extended seed dataset comprises a first subset of chemical objects that match a first property and a second subset of chemical objects that match a second property, and the instructions are executable to fine-tune the pretrained generative model using the first subset of chemical objects to obtain an intermediate generative model, and fine-tune the intermediate generative model using the second subset of chemical objects to obtain the fine-tuned generative model.

Another example provides a method of generating candidate molecules. The method comprises obtaining input of a seed dataset comprising one or more seed chemical objects corresponding to one or more seed molecules, each seed molecule comprising a value of a selected property within a selected range, inputting the one or more seed chemical objects into a neural network to form one or more seed chemical object embeddings, and performing a vector-based chemical similarity search by comparing the one or more seed chemical object embeddings to a plurality of embeddings stored in a vector database to obtain a search result dataset comprising a plurality of search result chemical objects, each embedding of the plurality of embeddings comprising a vector representation of a chemical object. The method further comprises forming an extended seed dataset based at least upon on the seed dataset and the search result dataset, fine-tuning a pretrained generative model using the extended seed dataset to form a fine-tuned generative model, using the fine-tuned generative model to generate a plurality of candidate chemical objects, and outputting the plurality of candidate chemical objects. In some such examples, each embedding of the embeddings stored in the vector database comprises embedded property information for the selected property, and the neural network is configured to form embeddings comprising embedded property information for the selected property. Additionally or alternatively, in some such examples, the method further comprises filtering the plurality of candidate chemical objects based on the selected range to form a filtered plurality of candidate chemical objects, and outputting the filtered plurality of candidate chemical objects. Additionally or alternatively, in some such examples, fine-tuning the pretrained generative model comprises fine-tuning an assembly of pretrained generative models to form an assembly of fine-tuned generative models, and using the fine-tuned generative model comprises using the assembly of fine-tuned generative models to generate the plurality of candidate chemical objects.

Another example provides a method of generating candidate molecules, the method comprising obtaining input of a seed dataset comprising one or more seed chemical objects and inputting the one or more seed chemical objects into a neural network to form one or more seed chemical object embeddings. The method further comprises performing a vector-based chemical similarity search by comparing the one or more seed chemical object embeddings to a plurality of embeddings stored in a vector database to obtain a search result dataset comprising a plurality of search result chemical objects, each embedding of the plurality of embeddings comprising a vector representation of a chemical object. The method further comprises forming an extended seed dataset based at least upon on the seed dataset and the search result dataset, and fine-tuning a pretrained generative model using the extended seed dataset to form a fine-tuned generative model

It will be understood that the configurations and/or approaches described herein are exemplary in nature, and that these specific embodiments or examples are not to be considered in a limiting sense, because numerous variations are possible. The specific routines or methods described herein may represent one or more of any number of processing strategies. As such, various acts illustrated and/or described may be performed in the sequence illustrated and/or described, in other sequences, in parallel, or omitted. Likewise, the order of the above-described processes may be changed.

The subject matter of the present disclosure includes all novel and non-obvious combinations and sub-combinations of the various processes, systems and configurations, and other features, functions, acts, and/or properties disclosed herein, as well as any and all equivalents thereof.

## Claims

1. A method (200) of generating candidate molecules, the method comprising:
obtaining (202) input of a seed dataset comprising one or more seed chemical objects corresponding to one or more seed molecules;
inputting (206) the one or more seed chemical objects into a neural network to form one or more seed chemical object embeddings;
performing (208) a vector-based chemical similarity search by comparing the one or more seed chemical object embeddings to a plurality of embeddings stored in a vector database to obtain a search result dataset comprising a plurality of search result chemical objects, each embedding of the plurality of embeddings comprising a vector representation of a chemical object;
forming (212) an extended seed dataset based at least upon on the seed dataset and the search result dataset; and
fine-tuning (216) a pretrained generative model using the extended seed dataset to form a fine-tuned generative model.

2. The method of claim 1, further comprising using the fine-tuned generative model to generate a plurality of candidate chemical objects, and outputting the plurality of candidate chemical objects, and
optionally, wherein fine-tuning the pretrained generative model comprises fine-tuning two or more pretrained generative models to form a corresponding two or more fine-tuned generative models, and wherein using the fine-tuned generative model comprises using the two or more fine-tuned generative models to generate the plurality of candidate chemical objects.

3. The method of claim 2, further comprising filtering the plurality of candidate chemical objects based on one or more of novelty, a chemical property, or a physical property to form filtered plurality of candidate chemical objects, and
optionally, further comprising performing further fine-tuning of the fine-tuned generative model using the filtered plurality of candidate chemical objects to form a further fine-tuned generative model, and using the further fine-tuned generative model to generate additional candidate chemical objects.

4. The method of claim 3, further comprising storing an embedding of at least one filtered candidate chemical object of the filtered plurality of candidate chemical objects in the vector database.

5. The method of any of claims 1 to 4, wherein each of the seed molecules comprises a value of a selected property within a selected range, and
optionally, wherein the method further comprises filtering the extended seed dataset to remove search result chemical objects corresponding to molecules comprising values outside the selected range for the selected property prior to fine-tuning the pretrained generative model.

6. The method of any of claims 1 to 5, wherein the extended seed dataset comprises a first subset of chemical objects that match a first property and a second subset of chemical objects that match a second property, and wherein fine-tuning the pretrained generative model comprises fine-tuning the pretrained generative model using the first subset of chemical objects to obtain an intermediate generative model, and fine-tuning the intermediate generative model using the second subset of chemical objects to obtain the fine-tuned generative model.

7. The method of any of claims 1 to 6, wherein the embeddings stored in the vector database comprises embedded property information.

8. A computing system (104, 300) implementing a neural network (110) configured to form embeddings of chemical objects, a vector database (108) storing embeddings of chemical objects, and one or more generative models (112), the computing system comprising:
a logic subsystem (302); and
a storage subsystem (304) comprising instructions executable by the logic subsystem to
input (206) one or more seed chemical objects into the neural network (110) to form one or more seed chemical object embeddings;
perform (208) a vector-based chemical similarity search by comparing the one or more seed chemical object embeddings to a plurality of embeddings stored in the vector database (108) to obtain a plurality of search result chemical objects, each embedding of the plurality of embeddings comprising a vector representation of a chemical object;
form (212) an extended seed dataset (124) based at least upon on the one or more seed chemical objects and the search result chemical objects; and
fine-tune (216) a pretrained generative model (132A) using the extended seed dataset to form a fine-tuned generative model (132B).

9. The computing system of claim 8, wherein the instructions are further executable to use the fine-tuned generative model to generate a plurality of candidate chemical objects, and output the plurality of candidate chemical objects.

10. The computing system of claim 9, wherein the instructions executable to fine-tune the pretrained generative model are executable to fine-tune two or more pretrained generative models to form a corresponding two or more fine-tuned generative models, and wherein the instructions are further executable to use the two or more fine-tuned generative models to generate the plurality of candidate chemical objects.

11. The computing system of either of claims 9 or 10, wherein the instructions are further executable to filter the plurality of candidate chemical objects based on one or more of novelty, a chemical property, or a physical property to form filtered plurality of candidate chemical objects, and
optionally, wherein the instructions are further executable to perform further fine-tuning of the fine-tuned generative model using the filtered plurality of candidate chemical objects to form a further fine-tuned generative model, and use the further fine-tuned generative model to generate additional candidate chemical objects.

12. The computing system of any of claims 8 to 11, wherein the extended seed dataset comprises a first subset of chemical objects that match a first property and a second subset of chemical objects that match a second property, and wherein the instructions are executable to fine-tune the pretrained generative model using the first subset of chemical objects to obtain an intermediate generative model, and fine-tune the intermediate generative model using the second subset of chemical objects to obtain the fine-tuned generative model.

13. A method (200) of generating candidate molecules, the method comprising:
obtaining (202) input of a seed dataset comprising one or more seed chemical objects corresponding to one or more seed molecules, each seed molecule comprising a value of a selected property within a selected range;
inputting (206) the one or more seed chemical objects into a neural network to form one or more seed chemical object embeddings;
performing (208) a vector-based chemical similarity search by comparing the one or more seed chemical object embeddings to a plurality of embeddings stored in a vector database to obtain a search result dataset comprising a plurality of search result chemical objects, each embedding of the plurality of embeddings comprising a vector representation of a chemical object;
forming (212) an extended seed dataset based at least upon on the seed dataset and the search result dataset;
fine-tuning (216) a pretrained generative model using the extended seed dataset to form a fine-tuned generative model;
using (220) the fine-tuned generative model to generate a plurality of candidate chemical objects; and
outputting (220) the plurality of candidate chemical objects.

14. The method of claim 13, wherein each embedding of the embeddings stored in the vector database comprises embedded property information for the selected property, and wherein the neural network is configured to form embeddings comprising embedded property information for the selected property.

15. The method of either of claims 13 or 14, further comprising filtering the plurality of candidate chemical objects based on the selected range to form a filtered plurality of candidate chemical objects, and outputting the filtered plurality of candidate chemical objects, and
optionally, wherein fine-tuning the pretrained generative model comprises fine-tuning an assembly of pretrained generative models to form an assembly of fine-tuned generative models, and wherein using the fine-tuned generative model comprises using the assembly of fine-tuned generative models to generate the plurality of candidate chemical objects.
